# EUROPÄISCHE PATENTANMELDUNG

(11) **EP 1 384 468 A1**
(43) Veröffentlichungstag der Anmeldung: **28.01.2004**
(21) Anmeldenummer: 02291883.3
(22) Anmeldetag: 25.07.2002
(51) Int. Cl.: A61K 7/40

(54) **Verfahren zum Schutz und zur Modulation von Tight Junctions**

(71) Anmelder: Cognis France S.A., 31360 Saint-Martory (FR)
(72) Erfinder: Pauly, Gilles, 54000 Nancy (FR); Jeanmaire, Christine, 54000 Nancy (FR)
(74) Vertreter: Cabinet HERRBURGER

(57) **Zusammenfassung**

Vorgeschlagen wird ein Verfahren zur kosmetischen Behandlung zur Verbesserung der epidermalen Barrierefunktion der Haut, Kopfhaut und Schleimhaut, dadurch gekennzeichnet, dass man eine Zubereitung, enthaltend mindestens eine Substanz, welche eine Modulation von Tight Junctions bewirkt, und/oder eine Modulation von Occludin und/oder Claudinen und/oder eine Modulation der Zona occludens über die Proteine ZO-1, ZO-2 und/oder ZO-3 bewirkt, topisch aufträgt.

Des Weiteren wird die Verwendung einer Substanz, welche eine Modulation von Tight Junctions und/oder eine Modulation von Occludin und/oder Claudinen und/oder eine Modulation der Zona occludens über die Proteine ZO-1, ZO-2 und/oder ZO-3 bewirkt, zur Herstellung kosmetischer Mittel zur Verbesserung der epidermalen Barrierefunktion der Haut, Kopfhaut und Schleimhaut und die Verwendung dieser Substanz zur Herstellung kosmetischer Mittel zum Schutz gegen Hautalterung und die Verwendung der Substanz zur Herstellung kosmetischer Mittel zum Schutz gegen schädigende Einflüsse durch Umweltgifte und UV-Strahlung vorgeschlagen.

## Beschreibung

### Gebiet der Erfindung

Die Erfindung befindet sich auf dem Gebiet der kosmetischen Zubereitungen und betrifft ein kosmetisches Verfahren zur Verbesserung der epidermalen Barrierefunktion der Haut, Kopfhaut und Schleimhaut und zum Schutz der menschlichen Haut gegen die Alterung und gegen schädigende Einflüsse durch Umweltgifte und UV-Strahlung. Des weiteren betrifft die Erfindung die Verwendung einer Substanz, welche eine Modulation von Tight Junctions und/oder eine Modulation von Occludin und/oder Claudinen und/oder eine Modulation der Zona occludens über die Proteine ZO-1, ZO-2 und/oder ZO-3 bewirkt, zur Herstellung von kosmetischen Mitteln zur Verbesserung der epidermalen Barrierefunktion der Haut, Kopfhaut und Schleimhaut und zum Schutz gegen Hautalterung, toxische Umwelteinflüsse und UV-Strahlung.

### Stand der Technik

Tight Junctions - auch Occluding Junctions, Zonula occuladens oder Schlussleisten genannt - verbinden benachbarte Zellen eines Gewebes an ihrem luminalen Ende miteinander. Sie trennen die Flüssigkeitsräume auf der luminalen und serosalen Oberfläche des Epithels voneinander ab und bilden eine Grenze zwischen apikaler (Zellseite zum Lumen) und basolateraler (Zellkörper und -basis) Membran der Einzelzelle.

Tight Junctions erfüllen zwei wesentliche Funktionen:

Sie verhindern die Passage von Molekülen und Ionen durch die Zwischenräume von Zellen und schützen so Zellen vor schädigenden Substanzen, da Materialien, die durch Diffusion oder aktiven Transport die Zelle passieren müssen, kontrolliert aufgenommen werden.

Sie blockieren den Austausch oder den Fluß intrazellulärer Substanzen wie beispielsweise von Membranproteinen und tragen so zum Erhalt spezieller Funktionen wie Rezeptoraktivierte Endocytose oder Exocytose an der basolateralen Oberfläche bei.

Somit ermöglichen Tight Junctions auch die wichtige Aufrechterhaltung von Konzentrationsgradienten über Epithelien.

Tight Junctions bestehen aus quervernetzten, in der Membran verankerten Proteinsträngen.

Die Hauptkomponenten sind Proteine wie Occludin - ein Transmembran-Protein von 65 kDa -, ZO-1 (Zona occludens - 1), ZO-2, ZO-3, sowie die Gruppe der Claudine, von denen es mehr als 20 unterschiedliche Proteine gibt.

Lange Zeit nahm man an, dass Tight Junctions nur in einfachen Geweben wie beispielsweise im Epithel, das Blutgefäße abgrenzt, vorhanden sind. In der Zwischenzeit konnten jedoch auch Proteine wie Occludin, ZO-1 und ZO-2, die mit Tight Junctions assoziiert werden müssen, in der Haut nachgewiesen werden.

M. Furuse et al. haben bei einer Gruppe von Mäusen, die mit trockener, faltiger Haut verendeten, nachweisen können, dass durch das Fehlen von Claudinen in den Tight Junctions des Stratum granulosum die epidermale Barrierefunktion der Haut stark geschädigt war, die Zellen konnten kein Wasser halten [M. Furuse, M. Hata, K. Furuse et al. The Journal of Cell Biology, 156 (no.6): 1099-1111, 2002].

Auch an der Entstehung einiger Krankheiten wie entzündlicher Darmerkrankungen IBD (Inflammatory Bowel Disease) und Schuppenflechte (Psoriasis) sind Fehlfunktionen der Tight Junctions beteiligt. In Untersuchungen zur Schuppenflechte wurde durch immunhistologische Bestimmungen mit Antikörpern gegen Proteine in den Tight Junctions deutliche Unterschiede in der Lokalisation der Proteine zwischen gesunder und pathologischer Haut festgestellt.

In der Internationalen Patentanmeldung **WO 99/35166** werden cyclische Peptide, die die Occludin gesteuerte Zelladhesion beeinflussen, sowie pharmazeutische Zubereitungen, die diese Peptide enthalten, offenbart. Unter anderem werden Methoden zur Erhöhung der Vasopermeabilität, zur Reduzierung der Zelladhesion, zur Absorptionsverbesserung von Arzneistoffen, insbesondere von Cytostatika oder Aufnahme von Arzneistoffen in das Zentralnervensystem, sowie eine Methode zur Verminderung der Angiogenese beschrieben, die diesen Mechanismus der Modulation Occludin-gesteuerter Zelladhesion ausnutzen. Ebenso wird ein Verfahren zur Detektion Occludin-gesteuerter Zellen beschrieben.

Die Aufgabe der vorliegenden Patentanmeldung hat darin bestanden, neue Mechanismen zur Verbesserung der Barrierefunktion der Haut, Kopfhaut und Schleimhaut zu finden, die zu einer Verzögerung der Hautalterung und zu einem Schutz der Haut, Kopfhaut und Schleimhaut gegen Umwelteinflüsse, wie toxische Substanzen oder UV-Strahlung beitragen und somit effektiv in kosmetischen Zubereitungen für die dermale Anwendung genutzt werden können.

### Beschreibung der Erfindung

Gegenstand der Erfindung sind Verfahren zur kosmetischen Behandlung zur Verbesserung der epidermalen Barrierefunktion der Haut, Kopfhaut und Schleimhaut, dadurch gekennzeichnet, dass man eine Zubereitung, enthaltend mindestens eine Substanz, welche eine Modulation von Tight Junctions bewirkt, und/oder eine Modulation von Occludin und/oder Claudinen und/oder eine Modulation der Zona occludens über die Proteine ZO-1, ZO-2 und/oder ZO-3 bewirkt, topisch aufträgt.

Weitere Gegenstände der Erfindung sind die Verwendung einer Substanz, welche eine Modulation von Tight Junctions und/oder eine Modulation von Occludin und/oder Claudinen und/oder eine Modulation der Zona occludens über die Proteine ZO-1, ZO-2 und/oder ZO-3 bewirkt, zur Herstellung kosmetischer Mittel zur Verbesserung der epidermalen Barrierefunktion der Haut, Kopfhaut und Schleimhaut, die Verwendung dieser Substanz zur Herstellung kosmetischer Mittel zum Schutz gegen Hautalterung und die Verwendung der Substanz zur Herstellung kosmetischer Mittel zum Schutz gegen schädigende Einflüsse durch Umweltgifte und UV-Strahlung vorgeschlagen.

Überraschenderweise wurde gefunden, dass die Modulation von Tight Junctions, insbesondere über die Proteine der Tight Junctions zu einer Erhaltung und Verbesserung der epidermalen Barrierefunktion der Haut, Kopfhaut und Schleimhaut führt. Diese Funktion ist eine essentielle Voraussetzung nicht nur für die Gesundheit, sondern auch für die Kosmetik. Das topische Auftragen einer Zubereitung, enthaltend eine Tight-Junctions modulierende Substanz, auf Haut, Schleimhaut oder Kopfhaut, vorzugsweise Haut und insbesondere trockene oder alternde Haut bewirkt durch die verbesserte Barrierefunktion einen verringerten Verlust an Feuchtigkeit. Sie schützt auf diese Weise nicht nur die Haut gegen Alterung, sondern auch gegen den schädigenden Einfluß von UV-Strahlung. Eine bessere Barriere trägt außerdem dazu bei, dass Umweltgifte nicht in die Zellen aufgenommen werden.

Somit haben die topisch applizierten kosmetischen Mittel, die mindestens eine Substanz enthalten, welche die Modulation von Tight Junctions bewirkt, einen vorbeugenden Effekt gegen Hautalterung und schädigende Einflüsse durch Umweltgifte und UV-Strahlung.

Die Modulation der Tight Junctions führt jedoch auch zu einer Verbesserung der epidermalen Barrierefunktion nach einer bereits eingetretenen Schädigung und bietet somit der Haut, Kopfhaut und Schleimhaut Gelegenheit sich schneller zu regenerieren.

Die Modulation der Tight Junctions wird vorzugsweise über die Modulation der Proteine Occludin und/oder über Claudine und/oder über die Proteine ZO-1, ZO-2 und/oder ZO-3 bewirkt, dazu können Peptide eingesetzt werden oder Pflanzenextrakte.

Möglich ist ebenfalls eine Modulation der Tight Junctions über mindestens eine Substanz, die ausgewählt ist aus der Gruppe, die gebildet wird von Modulatoren der Differentierung, Calciummodulatoren, Wachstumsfaktoren wie EGF (epidermal growth factor), TNF alpha (Tumor necrosis factor alpha), Cytokinen wie Interferon gamma, Transkriptionsfaktoren wie KLF4, Vitaminen, beispielweise Vitamin A und Vitamin D, Hormonen wie PTHrP (parathyroid hormone-related peptide), Transglutaminasen und Caspasen. Unter den Modulatoren der Differenzierung besonders bevorzugt ist der Einsatz von Calciummodulatoren.

Neben diesen Substanzen, Peptiden oder Pflanzenextrakten können die kosmetischen Mittel außerdem UV-Lichtschutzfaktoren und/oder Antioxidantien enthalten. Die Kombination aus Substanzen, die eine Modulation von Tight Junctions und/oder eine Modulation von Occludin und/oder Claudinen und/oder eine Modulation der Zona occludens über die Proteine ZO-1, ZO-2 und/oder ZO-3 bewirken mit UV-Lichtschutzfaktoren und/oder Antioxidantien führt durch die unterschiedlichen Mechanismen zu einer synergistischen Wirkungsweise und bietet einen hervorragenden Schutz gegen schädigende Einflüsse und Hautalterung durch UV-Lichteinwirkung.

### UV-Lichtschutzfilter und Antioxidantien

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
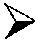 3-Benzylidencampher bzw. 3-Benzylidennorcampher und dessen Derivate, z.B. 3-(4-Methylbenzyliden)campher;
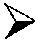 4-Aminobenzoesäurederivate, vorzugsweise 4-(Dimethylamino)benzoesäure-2-ethylhexylester, 4-(Dimethylamino)benzoesäure-2-octylester und 4-(Dimethylamino) benzoe-säureamylester;
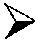 Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
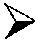 Ester der Salicylsäure, vorzugsweise Salicylsäure-2-ethylhexylester, Salicylsäure-4-isopropylbenzylester, Salicylsäurehomomenthylester;
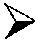 Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
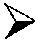 Ester der Benzalmalonsäure, vorzugsweise 4-Methoxybenzmalonsäuredi-2-ethylhexylester;
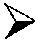 Triazinderivate, wie z.B. 2,4,6-Trianilino-(p-carbo-2'-ethyl-1'-hexyloxy)-1,3,5-triazin und Octyl Triazon, oder Dioctyl Butamido Triazone (Uvasorb® HEB);
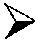 Propan-1,3-dione, wie z.B. 1-(4-tert.Butylphenyl)-3-(4'methoxyphenyl)propan-1,3-dion;
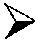 Ketotricyclo(5.2. 1 .0)decan-Derivate.

Als wasserlösliche Substanzen kommen in Frage:
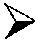 2-Phenylbenzimidazol-5-sulfonsäure und deren Alkali-, Erdalkali-, Ammonium-, Alkylammonium-, Alkanolammonium- und Glucammoniumsalze;
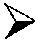 Sulfonsäurederivate von Benzophenonen, vorzugsweise 2-Hydroxy-4-methoxybenzophenon-5-sulfonsäure und ihre Salze;
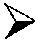 Sulfonsäurederivate des 3-Benzylidencamphers, wie z.B. 4-(2-Oxo-3-bornylidenmethyl)benzolsulfonsäure und 2-Methyl-5-(2-oxo-3-bomyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 1-Phenyl-3-(4'-isopropylphenyl)-propan-1,3-dion sowie Enaminverbindungen. Die UV-A und UV-B-Filter können selbstverständlich auch in Mischungen eingesetzt werden. Besonders günstige Kombinationen bestehen aus den Derivate des Benzoylmethans,, z.B. 4-tert.-Butyl-4'methoxydibenzoyimethan (Parsol® 1789) und 2-Cyano-3,3-phenylzimtsäure-2-ethylhexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden deartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-Apalmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

## Patentansprüche

1. Verfahren zur kosmetischen Behandlung zur Verbesserung der epidermalen Barrierefunktion der Haut, Kopfhaut und Schleimhaut, **dadurch gekennzeichnet, dass** man eine Zubereitung, enthaltend mindestens eine Substanz, welche eine Modulation von Tight Junctions bewirkt, topisch aufträgt.

2. Verfahren zur kosmetischen Behandlung zur Verbesserung der epidermalen Barrierefunktion der Haut, Kopfhaut und Schleimhaut, **dadurch gekennzeichnet, dass** man eine Zubereitung, enthaltend mindestens eine Substanz, welche eine Modulation von Occludin und/oder Claudinen bewirkt, topisch aufträgt.

3. Verfahren zur kosmetischen Behandlung zur Verbesserung der epidermalen Barrierefunktion der Haut, Kopfhaut und Schleimhaut, **dadurch gekennzeichnet, dass** man eine Zubereitung, enthaltend mindestens eine Substanz, welche eine Modulation der Zona occludens über die Proteine ZO-1, ZO-2 und/oder ZO-3 bewirkt, topisch aufträgt.

4. Verwendung einer Substanz, welche eine Modulation von Tight Junctions und/oder eine Modulation von Occludin und/oder Claudinen und/oder eine Modulation der Zona occludens über die Proteine ZO-1, ZO-2 und/oder ZO-3 bewirkt, zur Herstellung kosmetischer Mittel zur Verbesserung der epidermalen Barrierefunktion der Haut, Kopfhaut und Schleimhaut.

5. Verwendung einer Substanz, welche eine Modulation von Tight Junctions und/oder eine Modulation von Occludin und/oder Claudinen und/oder eine Modulation der Zona occludens über die Proteine ZO-1, ZO-2 und/oder ZO-3 bewirkt, zur Herstellung kosmetischer Mittel zum Schutz der Haut gegen Alterung.

6. Verwendung einer Substanz, welche eine Modulation von Tight Junctions und/oder eine Modulation von Occludin und/oder Claudinen und/oder eine Modulation der Zona occludens über die Proteine ZO-1, ZO-2 und/oder ZO-3 bewirkt, zur Herstellung kosmetischer Mittel zum Schutz der Haut, Kopfhaut und Schleimhaut gegen toxische Umwelteinflüsse.

7. Verwendung einer Substanz, welche eine Modulation von Tight Junctions und/oder eine Modulation von Occludin und/oder Claudinen und/oder eine Modulation der Zona occludens über die Proteine ZO-1, ZO-2 und/oder ZO-3 bewirkt, zur Herstellung kosmetischer Mittel zum Schutz der Haut, Kopfhaut und Schleimhaut gegen UV-Lichteinwirkung.

8. Verwendung gemäß mindestens einem der Ansprüche 4 bis 7, **dadurch gekennzeichnet, dass** die Substanz ein Peptid ist, welches eine Modulation von Tight Junctions und/oder eine Modulation von Occludin und/oder Claudinen und/oder eine Modulation der Zona occludens über die Proteine ZO-1, ZO-2 und/oder ZO-3 bewirkt.

9. Verwendung gemäß mindestens einem der Ansprüche 4 bis 7, **dadurch gekennzeichnet, dass** die Substanz ein Pflanzenextrakt ist, welcher eine Modulation von Tight Junctions und/oder eine Modulation von Occludin und/oder Claudinen und/oder eine Modulation der Zona occludens über die Proteine ZO-1, ZO-2 und/oder ZO-3 bewirkt.

10. Verwendung gemäß mindestens einem der Ansprüche 4 bis 7, **dadurch gekennzeichnet, dass** die Substanz ausgewählt ist aus der Gruppe, die gebildet wird von Modulatoren der Differenzierung, Calciummodulatoren, Wachstumsfaktoren wie EGF (epidermal growth factor), TNF alpha (Tumor necrosis factor alpha), Cytokinen wie Interferon gamma, Transkriptionsfaktoren wie KLF4, Vitaminen, Hormonen wie PTHrP (parathyroid hormone-related peptide), Transglutaminasen, Caspasen.

11. Kosmetische Zubereitungen, enthaltend mindestens eine Substanz, welche eine Modulation von Tight Junctions und/oder eine Modulation von Occludin und/oder Claudinen und/oder eine Modulation der Zona occludens über die Proteine ZO-1, ZO-2 und/oder ZO-3 bewirkt, und UV-Lichtschutzfaktoren und/oder Antioxidantien.
